# EUROPÄISCHE PATENTSCHRIFT

(11) **EP 0 672 654 B1**
(45) Veröffentlichungstag und Bekanntmachung des Hinweises auf die Patenterteilung: **10.09.1997**
(21) Anmeldenummer: 95103488.3
(22) Anmeldetag: 10.03.1995
(51) Int. Cl.: C07C 271/60, A01N 47/12

(54) **Fungizide Carbamoyloximcarbonsäureamide**
Fungicidal carbamoyloxime carbonic acid amides
Amides carbamoyleoxime carboxyliques fongicides

(30) Priorität: 19.03.1994 DE 4409432; 27.10.1994 DE 4438374
(43) Veröffentlichungstag der Anmeldung: 20.09.1995
(73) Patentinhaber: BASF AKTIENGESELLSCHAFT, 67056 Ludwigshafen (DE)
(72) Erfinder: Wagner, Oliver, Dr., D-66450 Bexbach (DE); Müller, Thomas, Dr., D-67258 Hessheim (DE); Eicken, Karl, Dr., D-67157 Wachenheim (DE); Wettling, Thomas, Dr., D-67117 Limburgerhof (DE); Ammermann, Eberhard, Dr., D-64646 Heppenheim (DE); Lorenz, Gisela, Dr., D-67434 Hambach (DE)

(56) Entgegenhaltungen:
- EP-A- 0 398 072
- EP-A- 0 493 683
- WO-A-91/16299

## Beschreibung

Die vorliegende Erfindung betrifft neue Carbamoyloximcarbonsäureamide der allgemeinen Formel I 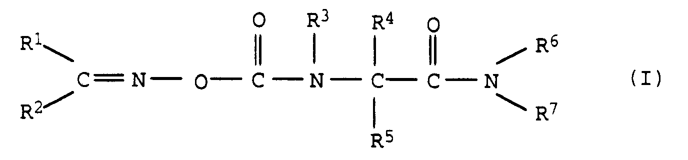 sowie deren Salze, in denen die Reste R¹ bis R⁷ die folgende Bedeutung haben:
- R¹: C₁-C₈-Alkyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₃-C₇-Cycloalkyl, C₅-C₇-Cycloalkenyl, Aryl und Heteroaryl, wobei die cyclischen Reste ihrerseits einen bis drei der folgenden Substituenten tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy,
C₂-C₈-Alkenyl oder C₂-C₈-Alkinyl, wobei diese Reste partiell oder vollständig halogeniert sein und/oder eine oder zwei der folgenden Gruppen tragen können: C₁-C₄-Halogenalkyl, C₁-C₄-Alkoxyalkyl, C₁-C₄-Alkoxy, C₁-C₄-Halogenalkoxy, Aryl und Aryloxy,
C₃-C₇-Cycloalkyl oder C₅-C₇-Cycloalkenyl, wobei diese Reste partiell oder vollständig halogeniert sein und/oder eine oder zwei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, Aryl und Aryl-(C₁-C₄)-alkyl, wobei in den Gruppen, welche Aryl enthalten dieses einen bis drei der folgenden Substituenten tragen kann: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy,
Aryl oder Heteroaryl, wobei diese Reste ein bis fünf Halogenatome und/oder eine bis drei der folgenden Gruppen tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy;
- R²: Wasserstoff oder einen der Reste R¹ oder
- R¹ und R²: gemeinsam mit dem Kohlenstoffatom das sie trägt einen Fluorenylrest, welcher eine bis drei der folgenden Gruppen tragen kann: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy oder
- R¹ und R²: gemeinsam mit dem Kohlenstoffatom das sie trägt einen 5- bis 8-gliedrigen gesättigten Ring, welcher als Ringglieder neben Kohlenstoff eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy und wobei Stickstoffatome als Heteroatome Wasserstoff oder eine C₁-C₄-Alkylgruppe tragen;
- R³: Wasserstoff, C₁-C₈-Alkyl oder C₃-C₇-Cycloalkyl, wobei diese Reste partiell oder vollständig halogeniert sein können;
- R⁴: C₁-C₈-Alkyl, C₃-C₇-Cycloalkyl oder Phenyl-(C₁-C₄)-alkyl, wobei der Phenylrest ein bis fünf Halogenatome und/oder eine bis drei der folgenden Gruppen tragen kann: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy;
- R⁵: Wasserstoff oder einen Rest R⁴ oder
- R⁴ und R⁵: gemeinsam mit dem C-Atom an das sie gebunden sind einen 3- bis 8-gliedrigen Ring, welcher neben Kohlenstoff als Ringglieder eines oder zwei der Heteroatome Sauerstoff, Schwefel und Stickstoff enthalten kann, wobei die Kohlenstoffatome des Rings eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy und C₁-C₄-Halogenalkoxy und wobei Stickstoffatome als Heteroatome Wasserstoff oder eine C₁-C₄-Alkylgruppe tragen;
- R⁶: Wasserstoff oder partiell oder vollständig halogeniertes C₁-C₈-Alkyl oder C₃-C₇-Cycloalkyl;
- R⁷: C₁-C₈-Alkyl oder C₂-C₈-Alkenyl, wobei diese Reste partiell oder vollständig halogeniert sein und/oder eine oder zwei der folgenden Gruppen tragen können: Cyano, Nitro, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl und Aryl und wobei die Arylgruppen ihrerseits einen bis drei der folgenden Substituenten tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy,
C₃-C₇-Cycloalkyl oder C₅-C₇-Cycloalkenyl, wobei diese Reste partiell oder vollständig halogeniert sein, und/oder eine oder zwei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio,
Aryl oder Heteroaryl, wobei diese Reste partiell oder vollständig halogeniert sein und/oder eine bis drei der folgenden Gruppen tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy oder
- R⁶ und R⁷: gemeinsam mit dem Stickstoffatom an das sie gebunden sind einen 4- bis 8-gliedrigen gesättigten Ring, wobei der Ring noch eines oder zwei der Heteroatome Sauerstoff, Schwefel und Stickstoff enthalten kann und die Kohlenstoffatome des Rings eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Heteroaryl und wobei die cyclischen Gruppen ein bis fünf Halogenatome und/oder einen bis drei der folgenden Substituenten tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy und wobei weitere Stickstoffatome als Heteroatome Wasserstoff oder eine C₁-C₄-Alkylgruppe tragen.

Außerdem betrifft die Erfindung Verfahren zur Herstellung der Verbindungen I, I enthaltende Mittel sowie ein Verfahren zur Bekämpfung von Schadpilzen und die Verwendung der Verbindungen I hierzu.

Aus der Literatur sind fungizide Carbamoylcarbonsäureamide wie das N-(tert.-Butoxycarbonyl)-L-valin-4-methoxyphenylethylamid 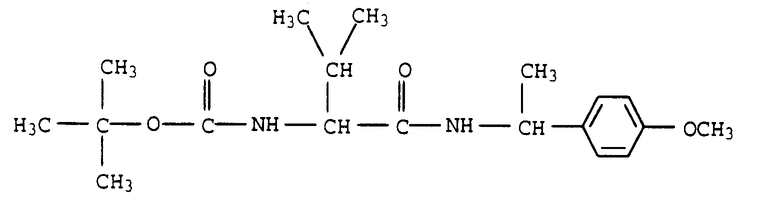 (vgl. EP-A-0 398 072) bzw. das N-(4-Methylbenzyloxycarbonyl)valin-[2-(3,4-dimethoxyphenyl)ethyl]methylamid 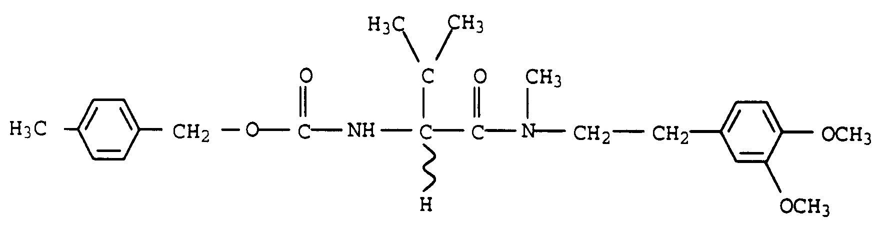 (vgl. EP-A-0 493 683) bekannt, die jedoch hinsichtlich ihrer Wirkung noch nicht befriedigen können.

Der vorliegenden Erfindung lagen daher neue Carbamoyloximcarbonsäureamide mit verbesserter Wirkung gegen Schadpilze als Aufgabe zugrunde.

Demgemäß wurden die eingangs definierten Verbindungen I gefunden.

Ferner wurden sie enthaltende Mittel, Verfahren zur Herstellung der Verbindungen I und der sie enthaltenden Mittel gefunden und desweiteren ein Verfahren zur Bekämpfung von Schadpilzen und die Verwendung der Verbindungen I hierzu.

Die Verbindungen I sind in an sich bekannter Weise aus den entsprechenden Aminosäuren II erhältlich und zwar bevorzugt nach den im folgenden beschriebenen Verfahren A bzw. B (die Literaturzitate "Houben-Weyl" beziehen sich auf: Houben-Weyl, Methoden der Organischen Chemie, 4. Auflage, Thieme Verlag, Stuttgart).

### Verfahren A

Die Carbamoyloximcarbonsäureamide I erhält man, indem man aus den N-geschützten Aminosäuren IIa, in denen G eine an sich bekannte Aminosäure-Schutzgruppe bedeutet, und den Aminen III die N-geschützten Aminosäureamide IV herstellt, aus diesen durch Abspalten der Schutzgruppe G die Aminosäureamide V freisetzt und diese mit den Chlorformyloximen VI in Gegenwart von Basen weiter umsetzt.

### Stufe Aa: Herstellung der N-geschützten Aminosäuren IIa

Die N-geschützten Aminosäuren IIa können in an sich bekannter Weise aus den entsprechenden Aminosäuren II hergestellt werden (vgl. auch "Houben-Weyl", Band 15/1, Seite 46 ff.).

Die Schutzgruppen G haben die Funktion, daß sie unerwünschte Reaktionen an den sie tragenden Aminogruppen verhindern; andererseits können sie mit geeigneten Methoden, z.B. durch saure Hydrolyse oder durch Hydrieren, in der Regel selektiv entfernt werden.

Bevorzugte Schutzgruppen G sind die Benzyloxycarbonylgruppe und vor allem die tert.-Butoxycarbonylgruppe.

Durch tert.-Butoxycarbonylgruppen geschützte Aminosäuren IIa erhält man beispielsweise durch Umsetzen der betreffenden Aminosäuren II mit Chlorameisensäure-tert.-butylester (vgl. "Houben-Weyl", Band 15/1, Seite 117 bis Seite 125).

Die Aminosäuren II ihrerseits sind bekannt oder können nach bekannten Methoden dargestellt werden (vgl. "Houben-Weyl", Band 15/1, Seite 46 bis Seite 305, vor allem Seite 117 bis Seite 125).

### Stufe Ab: Herstellung der N-geschützten Aminosäureamide IV

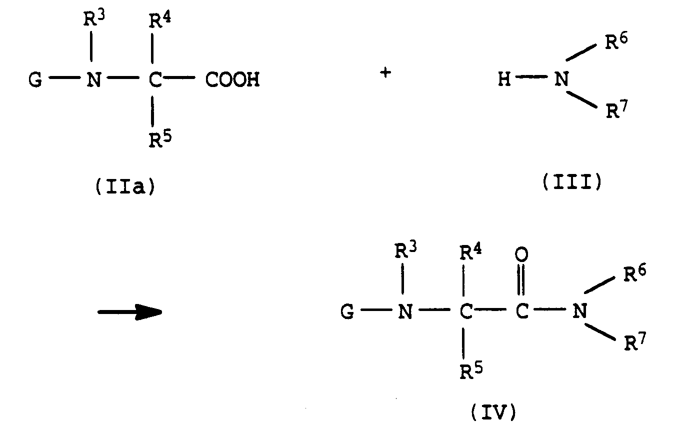

Die N-geschützten Aminosäureamide IV werden aus den N-geschützten Aminosäuren IIa und den Aminen III in an sich bekannter Weise hergestellt (vgl. "Houben-Weyl", Band 15/1, Seite 28 bis Seite 32).

Die Amine III sind ihrerseits bekannt oder können nach bekannten Methoden erhalten werden (vgl. Organikum, 15. Auflage, Deutscher Verlag der Wissenschaften, Berlin, 1977, Seite 610 ff.).

Vorzugsweise arbeitet man dabei so, daß man zunächst die N-geschützten Aminosäuren IIa in carboxyaktivierte Derivate, vor allem in Acylcyanide oder Anhydride, überführt (vgl. Tetrahedron Letters, Band 18, Seite 1595 bis Seite 1598 (1973) bzw. "Houben-Weyl", Band 15/1, Seite 28 bis Seite 32). Diese Derivate werden dann mit den Aminen III in Gegenwart von Basen zur Reaktion gebracht.

Zur Herstellung der carboxyaktivierten Acylcyanide eignet sich z.B. die Reaktion der N-geschützten Aminosäuren IIa mit Cyanphosphonsäurediethylester, vor allem in einem inerten Lösungsmittel wie Tetrahydrofuran oder Toluol.

Zur Herstellung der carboxyaktivierten Anhydride ist die Umsetzung der N-geschützten Aminosäuren IIa mit Kohlensäurechloriden wie Chlorameisensäure-iso-butylester in Gegenwart einer Base und gegegebenfalls in einem inerten Lösungsmittel wie Toluol oder Tetrahydrofuran bevorzugt.

Die Umsetzung der Amine III mit den carboxyaktivierten N-geschützten Aminosäuren IIa erfolgt vorzugsweise in einem Lösungsmittel wie Dichlormethan, Tetrahydrofuran oder Toluol.

Als Basen können insbesondere die Amine III selbst dienen, wobei man sie aus dem Rohprodukt üblicherweise zurückgewinnt.

In einer bevorzugten Ausführungsform dieser Verfahrensstufe (Ab) werden die N-geschützte Aminosäure IIa, das Amin III, das zur Erzeugung des caboxyaktivierten Derivates der N-geschützten Aminosäure IIa geeignete Reagens und die Base im Eintopfverfahren, gegebenenfalls in einem inerten Lösungsmittel, zur Reaktion gebracht, und das Rohprodukt wird anschließend in an sich bekannter Weise auf das N-geschützte Aminosäureamid IV aufgearbeitet.

### Stufe Ac: Herstellung der Aminosäureamide V

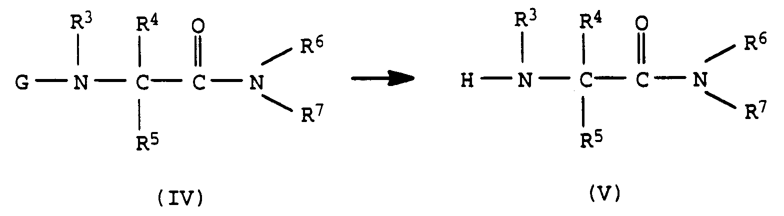

Aus den N-geschützten Aminosäureamiden IV werden durch Abspalten der Schutzgruppen) G die Aminosäureamide V hergestellt.

Diese Reaktion kann in an sich bekannter Weise durchgeführt werden (vgl. "Houben-Weyl", Band 15/1, Seite 46 bis Seite 305, vor allem Seite 126 bis Seite 129).

Im Falle der tert.-Butoxycarbonyl-Schutzgruppe erfolgt die Abspaltung beispielsweise unter Verwendung von Protonensäuren wie Salzsäure oder Trifluoressigsäure (loc. cit.).

### Stufe Ad: Herstellung der Carbamoyloximcarbonsäureamide I

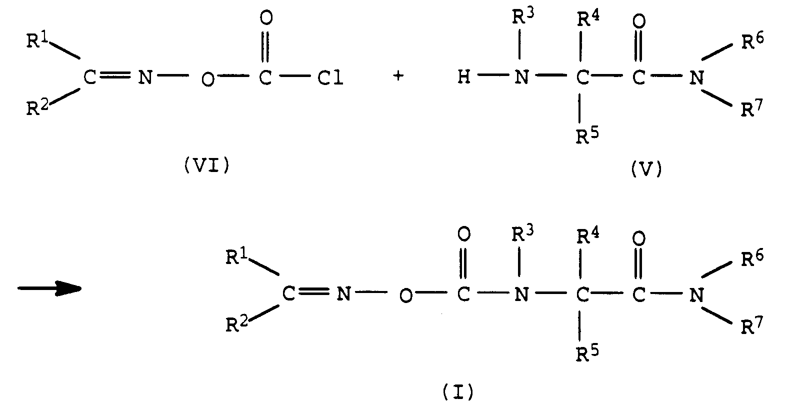

Die aus der Synthesestufe (Ac) resultierenden Aminosäureamide V werden in an sich bekannter Weise mit den Chlorformyloximen VI in Gegenwart von Basen in die Carbamoyloximcarbonsäureamide I überführt.

Die Chlorformyloxime VI sind bekannt oder können nach bekannten Verfahren, etwa durch Phosgenierung von Hydroxylaminen, hergestellt werden (vgl. z.B. Zeitschrift für Chemie, Band 9, Seite 344 bis 345 (1967)).

Die Umsetzung wird vorzugsweise in einem organischen Lösungsmittel oder Lösungsmittelgemisch durchgeführt. Bevorzugte Lösungsmittel sind Toluol, Methylenchlorid und Tetrahydrofuran.

Als Basen kommen anorganische und organische Basen gleichermaßen in Betracht, wobei organische Basen bevorzugt und hierunter wiederum tertiäre Amine wie Triethylamin, Pyridin und N-Methylpiperidin besonders bevorzugt sind.

Die Umsetzung wird in der Regel bei Temperaturen von (-40) bis 50, vorzugsweise bei (-10) bis 20°C durchgeführt.

### VERFAHREN B

Die Carbamoyloximcarbonsäureamide I lassen sich ferner herstellen, indem man die Aminosäuren II in Gegenwart von Basen mit den Chlorformyloximen VI zu den Carbamoyloximcarbonsäuren VII umsetzt und diese danach mit den Aminen III zur Reaktion bringt.

### Stufe Ba: Herstellung der Carbamoyloximcarbonsäuren VII

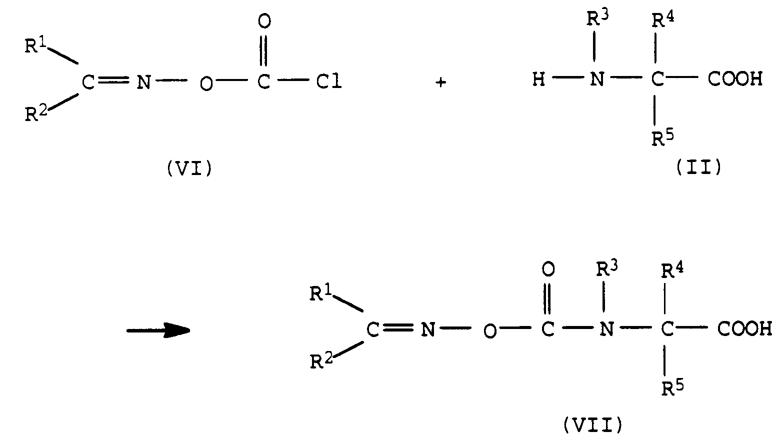

Man erhält die Carbamoyloximcarbonsäuren VII durch Umsetzen der Aminosäuren II mit den Chlorformyloximen VI in Gegenwart von Basen.

Als Basen eignen sich anorganische und organische Basen, unter denen organische Basen bevorzugt und tertiäre Amine wie Triethylamin, Pyridin und N-Methylpiperidin besonders bevorzugt sind.

Die Umsetzung wird vorzugsweise in einem organischen Lösungsmittel oder Lösungsmittelgemisch durchgeführt. Bevorzugte Lösungsmittel sind Toluol, Methylenchlorid und Tetrahydrofuran.

Die Reaktionstemperatur liegt in der Regel bei Temperaturen zwischen (-30) und 40, vorzugsweise bei (-10) und 20°C.

Die Durchführung dieser Reaktion ist dem Fachmann im übrigen geläufig, so daß von weitere Erläuterungen hierzu abgesehen werden kann (vgl. "Houben-Weyl", Band 15/1, Seite 117 bis Seite 125, bzw. Dev. Endocrinol., 13 (Neurohypophyseal Pept. Horm. Other Biol. Act. Pept.), Seite 37 bis Seite 47 (1981)).

### Stufe Bb: Herstellung der Carbamoyloximcarbonsäureamide I

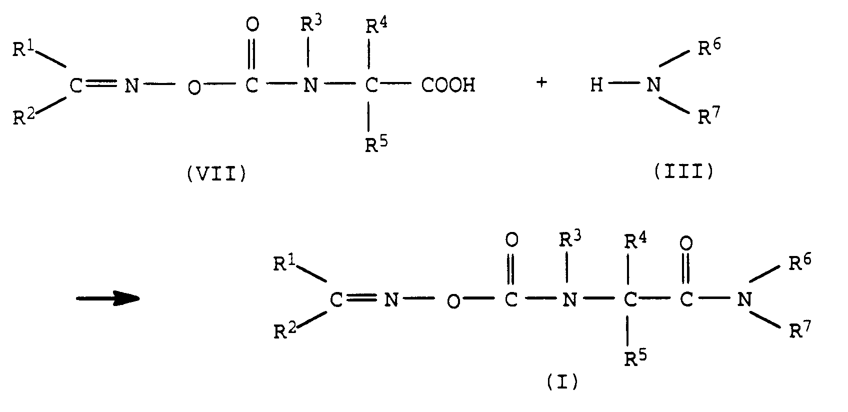

Man erhält die Carbamoyloximcarbonsäureamide I, indem man die entsprechenden Carbamoyloximcarbonsäuren VII mit den jeweiligen Aminen III umsetzt.

Vorzugsweise arbeitet man so, daß man zunächst die Carbamoyloximcarbonsäuren VII in carboxyaktivierte Derivate, vor allem in Acylcyanide oder Anhydride, überführt und im übrigen wie oben beim Verfahrensschritt (Ab) verfährt.

Die nach den Verfahren A bzw. B erhaltenen Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit werden können. Sofern die Zwischen- und Endprodukte als Feststoffe erhalten werden, kann die Reinigung auch beispielsweise durch Umkristallisieren oder Digerieren erfolgen.

Die Verbindungen der Formel I können gegebenenfalls in Abhängigkeit von der Art der Substituenten als geometrische und/oder optische Isomere oder Isomerengemische vorliegen. Sowohl die reinen Isomeren als auch die Gemische der Isomeren weisen die fungizide Wirkung auf.

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen freizuhaltenden Pflanzen, Flächen, Materialien oder Räume nicht schädigen und die Wirkung der Verbindungen I nicht beeinträchtigen.

Die Salze der Verbindungen I sind in an sich bekannter Weise zugänglich, vor allem durch Umsetzen der entsprechenden Carbamoyloximcarbonsäureamide I mit den genannten Säuren in Wasser oder einem inerten organischen Lösungsmittel bei Temperaturen von -80 bis 120°C, vorzugsweise 0 bis 60°C.

Bei der eingangs angegebenen Definition der Verbindungen I wurden Sammelbegriffe verwendet, die repräsentativ für die folgenden Substituenten stehen:
Halogen: Fluor, Chlor, Brom und Jod;
Alkyl: geradkettige oder verzweigte Alkylgruppen mit 1 bis 8 Kohlenstoffatomen, z.B. C₁-C₆-Alkyl wie Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 2,2-Dimethylpropyl, 1,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl bzw. partiell oder vollständig halogeniertes Alkyl: geradkettige oder verzweigte Alkylgruppen mit 1 bis 4 bzw. 8 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkoxygruppen mit 1 bis 4 Kohlenstoffatomen, z.B. C₁-C₃-Alkoxy wie Methyloxy, Ethyloxy, Propyloxy und 1-Methylethyloxy;
Alkoxyalkyl: geradkettige oder verzweigte Alkylgruppen mit 1 bis 8 Kohlenstoffatomen (wie vorstehend genannt), welche in einer beliebigen Position eine geradkettige oder verzweigte Alkoxygruppe (wie vorstehend genannt) mit im Falle von C₁-C₄-Alkoxyalkyl 1 bis 4 Kohlenstoffatomen tragen, wie Methoxymethyl, Ethoxymethyl, n-Propoxymethyl, n-Butoxymethyl, 1-Methoxyethyl, 2-Methoxyethyl, 1-Ethoxyethyl, 2-Ethoxyethyl, 2-n-Propoxyethyl und 2-Butoxyethyl;
Halogenalkoxy: geradkettige oder verzweigte Alkoxygruppen mit 1 bis 4 Kohlenstoffatomen (wie vorstehend genannt), wobei in diesen Gruppen die Wasserstoffatome partiell oder vollständig durch Halogenatome (wie vorstehend genannt) ersetzt sein können, z.B. C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind, z.B. C₁-C₄-Alkylthio wie Methylthio, Ethylthio, Propylthio, 1-Methylethylthio, n-Butylthio und tert.-Butylthio;
Alkoxycarbonyl: geradkettige oder verzweigte Alkoxygruppen mit 1 bis 4 C-Atomen (wie vorstehend genannt), welche über eine Carbonylgruppe (-CO-) an das Gerüst gebunden sind;
Alkenyl: geradkettige oder verzweigte Alkenylgruppen mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 2-Methyl-1-propenyl, 1-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Alkinylgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1methyl-2-propinyl;
Cycloalkyl: monocyclische Alkylgruppen mit 3 bis 7 Kohlenstoffringgliedern, z.B. C₃-C₇-Cycloalkyl wie Cyclopropyl, Cyclobutyl, Cyclopentyl, Cyclohexyl und Cycloheptyl;
Cycloalkenyl: monocyclische Alkylgruppen mit 5 bis 7 Kohlenstoffringgliedern die eine oder mehrere Doppelbindungen enthalten z.B. C₅-C₇-Cycloalkenyl wie Cyclopentenyl, Cyclohexenyl und Cycloheptenyl;
nicht-aromatische 4- bis 8-gliedrigen Ringe, welcher als Ringglieder neben Kohlenstoff noch ein oder zwei Sauerstoff-, Schwefel- oder Stickstoffatome enthalten wie gesättigte 5- oder 6-gliedrige Ringe mit 1 oder 2 Stickstoff- und/oder Sauerstoffatomen wie 3-Tetrahydrofuranyl, 1-Piperidinyl, 2-Piperidinyl, 3-Piperidinyl, 4-Piperidinyl, 2-Tetrahydropyranyl, 3-Tetrahydropyranyl, 4-Tetrahydropyranyl, 2-Morpholinyl und 3-Morpholinyl;
Aryl: monocyclische oder polycyclische aromatische Gruppen mit 6 bis 10 C-Atomen wie Phenyl und Naphthyl;
Arylalkyl: Arylgruppen (wie vorstehend genannt), welche im Falle von Aryl-(C₁-C₄)-alkyl über Alkylgruppen mit 1 bis 4 Kohlenstoffatomen (wie vorstehend genannt) an das Gerüst gebunden sind, z.B. Phenyl-(C₁-C₄)-alkyl wie Benzyl, 2-Phenylethyl, 3-Phenylpropyl, 4-Phenylbutyl, 1-Phenylethyl, 1-Phenylpropyl und 1-Phenylbutyl;
Aryloxy: Arylgruppen (wie vorstehend genannt), welche über ein Sauerstoffatom (-O-) an das Gerüst gebunden sind wie Phenoxy, 1-Naphthoxy und 2-Naphthoxy;
Heteroaryl: aromatische mono- oder polycyclische Reste, welche neben Kohlenstoffringgliedern zusätzlich 1 bis 4 Stickstoffatome oder 1 bis 3 Stickstoffatome und ein Sauerstoff- oder ein Schwefelatom oder ein Sauerstoff- oder ein Schwefelatom enthalten können, z.B.:
   - 5-gliedriges Heteroaryl, enthaltend 1 bis 3 Stickstoffatome: 5-Ring-Heteroarylgruppen, welche neben Kohlenstoffatomen 1 bis 3 Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
   - 5-gliedriges Heteroaryl, enthaltend 1 bis 4 Stickstoffatome oder 1 bis 3 Stickstoffatome und 1 Schwefelatom oder Sauerstoffatom oder 1 Sauerstoff- oder 1 Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
   - benzokondensiertes 5-gliedriges Heteroaryl, enthaltend 1 bis 3 Stickstoffatome oder 1 Stickstoffatom und/oder ein Sauerstoff- oder Schwefelatom: 5-Ring-Heteroarylgruppen, welche neben Kohlenstoffatomen 1 bis 4 Stickstoffatome oder 1 bis 3 Stickstoffatome und 1 Schwefel- oder Sauerstoffatom oder 1 Sauerstoff- oder ein Schwefelatom als Ringglieder enthalten können, und in welchen 2 benachbarte Kohlenstoffringglieder oder 1 Stickstoff- und 1 benachbartes Kohlenstoffringglied durch eine Buta-1,3-dien-1,4-diylgruppe verbrückt sein können;
   - über Stickstoff gebundenes 5-gliedriges Heteroaryl, enthaltend 1 bis 4 Stickstoffatome, oder über Stickstoff gebundenes benzokondensiertes 5-gliedriges Heteroaryl, enthaltend 1 bis 3 Stickstoffatome: 5-Ring-Heteroarylgruppen, welche neben Kohlenstoffatomen 1 bis 4 Stickstoffatome bzw. 1 bis 3 Stickstoffatome als Ringglieder enthalten können, und in welchen 2 benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend 1 bis 3 bzw. 1 bis 4 Stickstoffatome: 6-Ring-Heteroarylgruppen, welche neben Kohlenstoffatomen 1 bis 3 bzw. 1 bis 4 Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
   - benzokondensiertes 6-gliedriges Heteroaryl, enthaltend 1 bis 4 Stickstoffatome: 6-Ring-Heteroarylgruppen, in welchen 2 benachbarte Kohlenstoffringglieder durch eine Buta-1,3-dien-1,4-diylgruppe verbrückt sein können, z.B. Chinolin, Isochinolin, Chinazolin und Chinoxalin.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung gegen Schadpilze sind Verbindungen I bevorzugt, in denen die Reste in den folgenden Bedeutungen, und zwar für sich allein oder in Kombination bevorzugt sind:
- R¹: C₃-C₆-Cycloalkyl, wobei dieser Rest partiell oder vollständig halogeniert sein kann,
- R¹: C₁-C₈-Alkyl, wobei dieser Rest partiell oder vollständig halogeniert sein kann,
- R¹: Wasserstoff,
- R²: Aryl, wobei dieser Rest eine bis drei der folgenden Gruppen tragen kann: Halogen, C₁-C₄-Alkyl und C₁-C₄-Alkoxy,
- R²: C₁-C₈-Alkyl, wobei dieser Rest ein bis drei Halogenatome bzw. C₁-C₄-Alkoxygruppen tragen kann,
- R²: C₃-C₆-Cycloalkyl, wobei dieser Rest ein bis drei Halogenatome, C₁-C₄-Alkyl- und C₁-C₄-Alkoxygruppen tragen kann,
- R³: C₁-C₄-Alkyl,
- R³: Wasserstoff,
- R⁴: C₁-C₆-Alkyl und vor allem C₁-C₄-Alkyl,
- R⁴: C₃-C₆-Cycloalkyl, besonders bevorzugt Cyclopropyl, Cyclopentyl oder Cyclohexyl,
- R⁵: C₁-C₆-Alkyl, vor allem C₁-C₄-Alkyl,
- R⁵: Wasserstoff,
- R⁵: C₃-C₆-Cycloalkyl, vor allem Cyclopropyl, Cyclopentyl oder Cyclohexyl,
- R⁴: C₁-C₆-Alkyl und R⁵ Wasserstoff,
- R⁴: C₃-C₆-Cycloalkyl und R⁵ Wasserstoff,
- R⁶: C₁-C₄-Alkyl, wobei Methyl und Ethyl besonders bevorzugt sind,
- R⁶: Wasserstoff,
- R⁶: C₃-C₆-Cycloalkyl, vor allem Cyclopropyl,
- R⁷: C₃-C₇-Cycloalkyl, wobei dieser Rest durch eine bis zwei der folgenden Gruppen substituiert sein kann: Halogen, C₁-C₄-Alkyl, C₁-C₄-Halogenalkyl, C₁-C₄-Alkoxy und C₁-C₄-Halogenalkoxy; insbesondere Cyclopropyl, Cyclopentyl oder Cyclohexyl.

Außerdem sind Verbindungen I bevorzugt, in denen R⁷ durch Phenyl substituiertes C₁-C₄-Alkyl bedeutet, wobei der Alkylteil und der Phenylteil jeweils unsubstituiert sein oder bis zu zwei der folgenden Gruppen tragen können: Halogen, Cyano, Nitro, C₁-C₄-Alkyl (außer im Alkylteil), C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Phenyl und Phenoxy (außer im Alkylteil), insbesondere Benzyl, 1-Phenylethyl, 2-Phenylethyl, 1-Phenylpropyl, 2-Phenylpropyl, 3-Phenylpropyl, 1-Phenyl-iso-propyl, 2-Phenyl-iso-propyl, 1-Phenyl-n-butyl, 1-Phenyl-iso-butyl, 1-Phenyl-sec.-butyl, wobei der Alkylteil in diesen Gruppen einen der zwei der folgenden Substituenten tragen kann: Halogen, Cyano und C₁-C₄-Alkoxy und der Phenylteil einen oder zwei Substituenten aus: Fluor, Chlor, Brom, Nitro, Cyano, C₁-C₄-Alkyl, Trifluormethyl, C₁-C₄-Alkoxy oder Trifluormethoxy.

Ganz besonders bevorzugt sind im Hinblick auf ihre Verwendung die in den anschließenden Tabellen, vor allem Tabelle 4, zusammengestellten Verbindungen I.

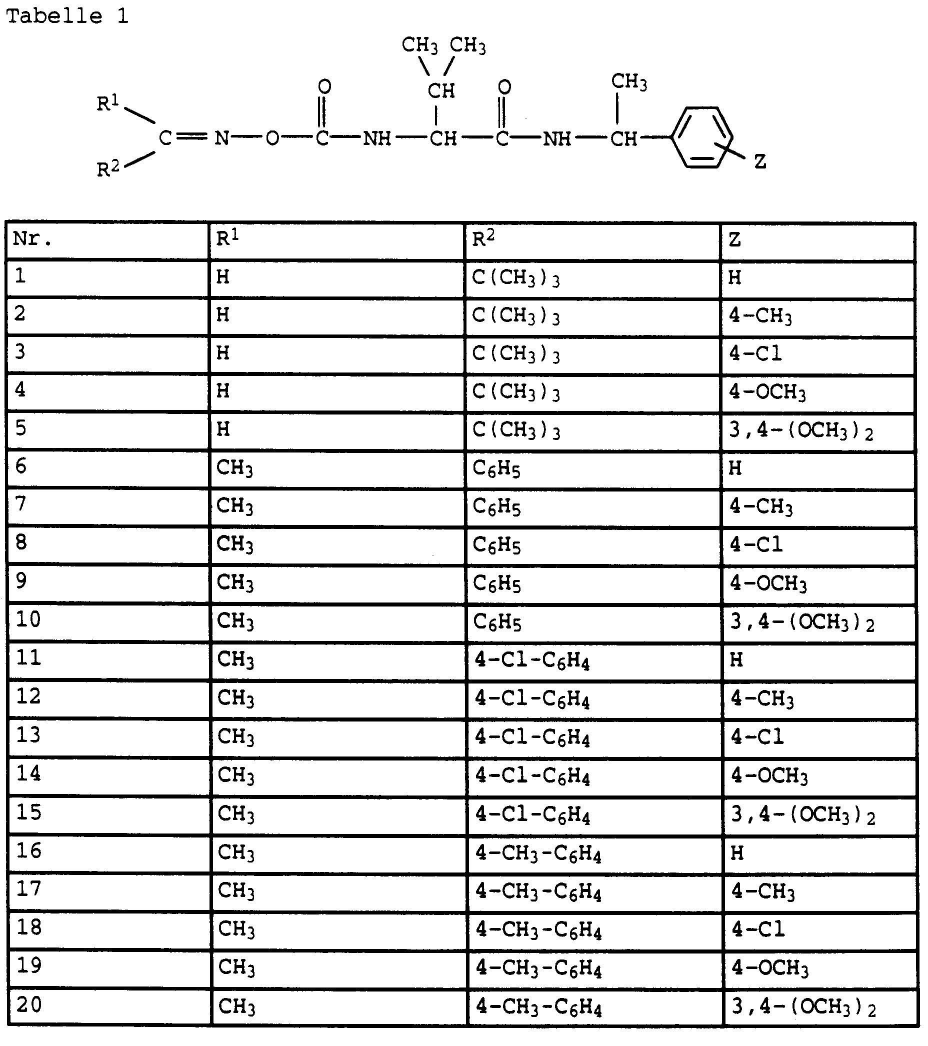

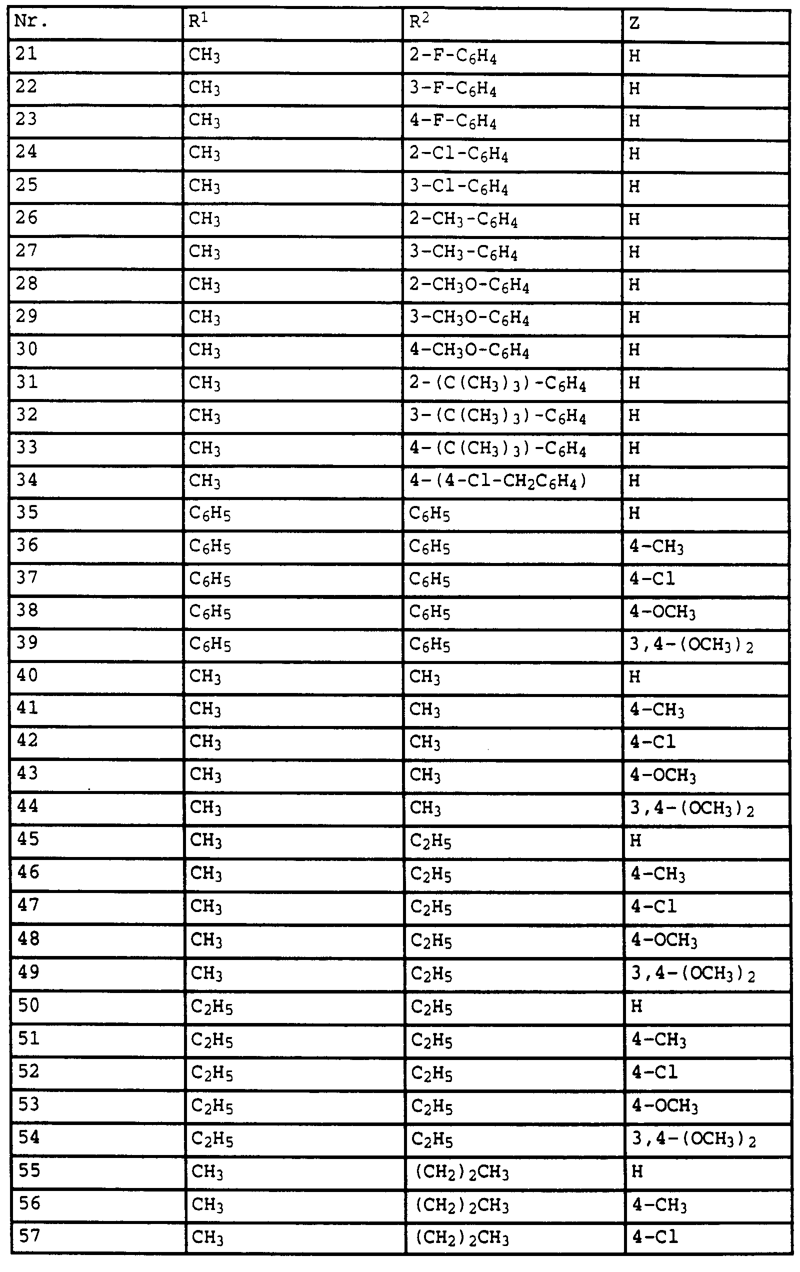

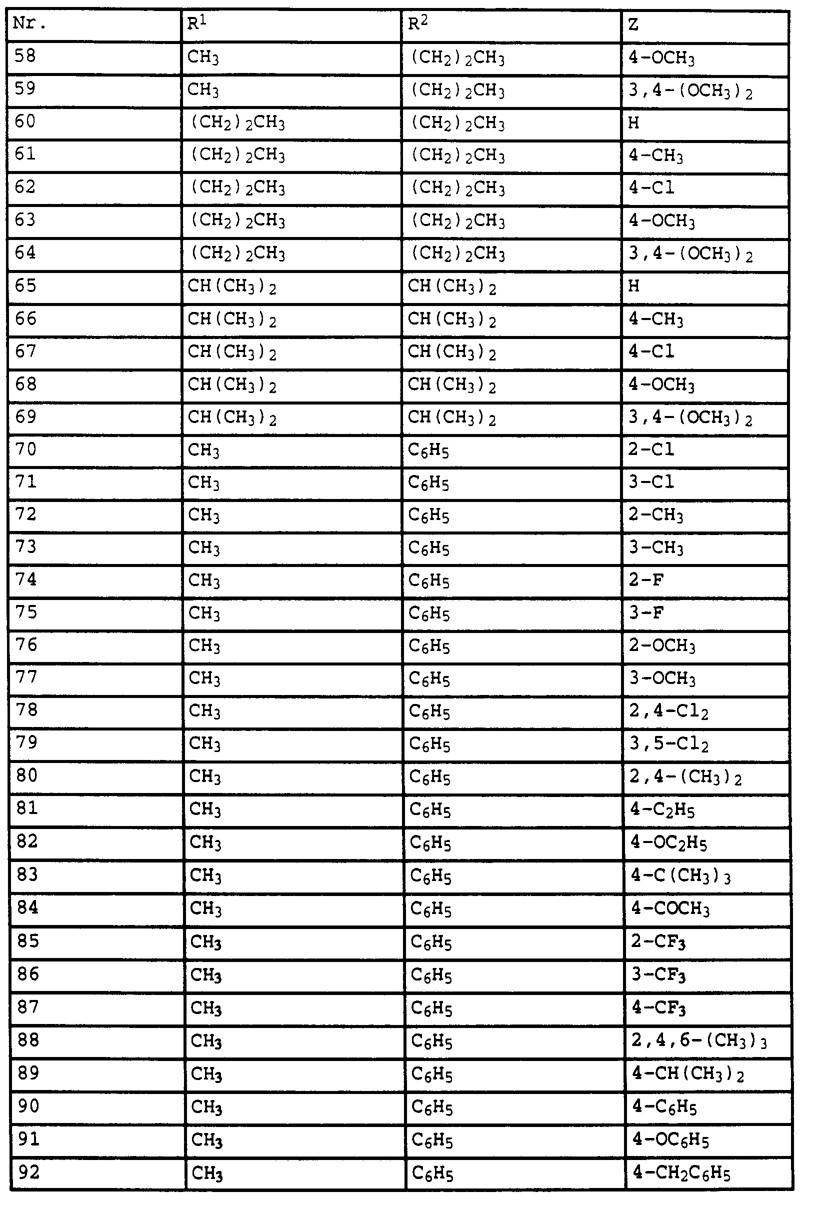

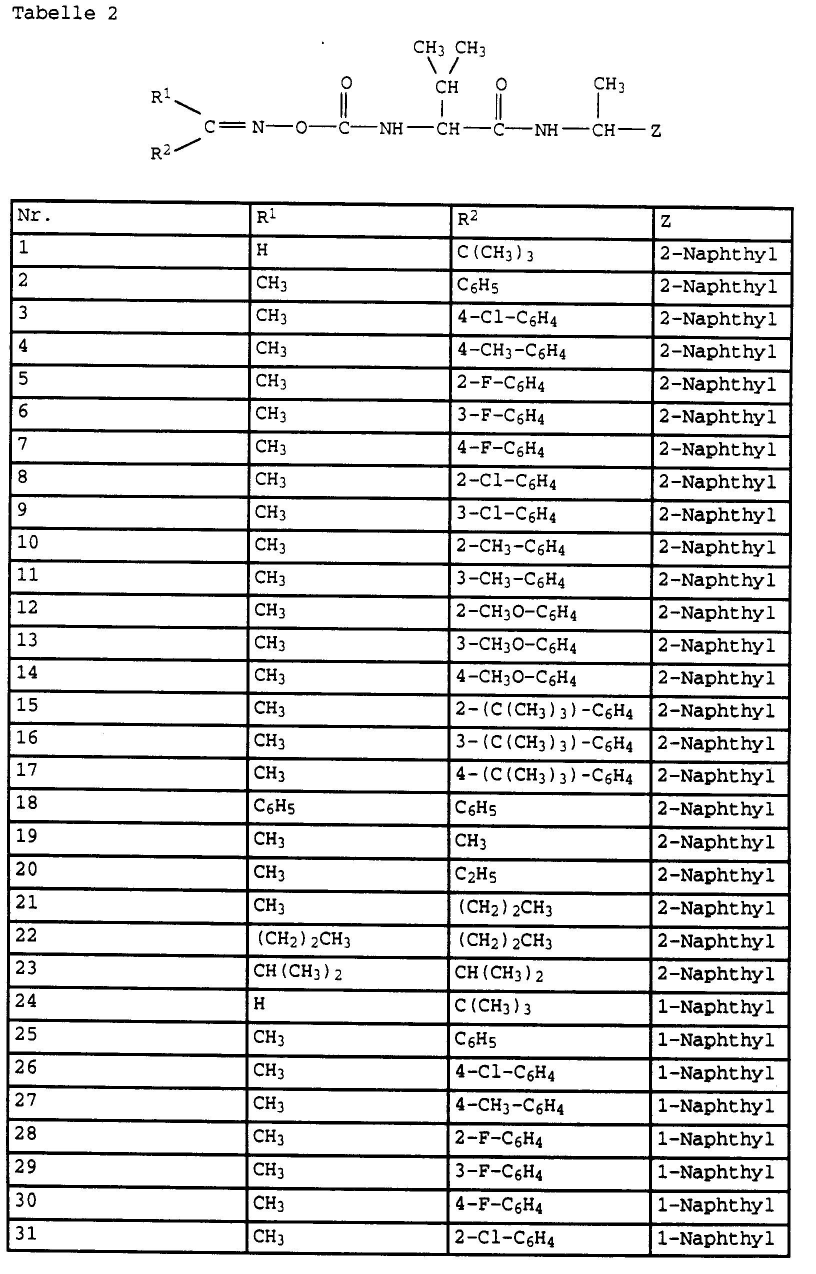

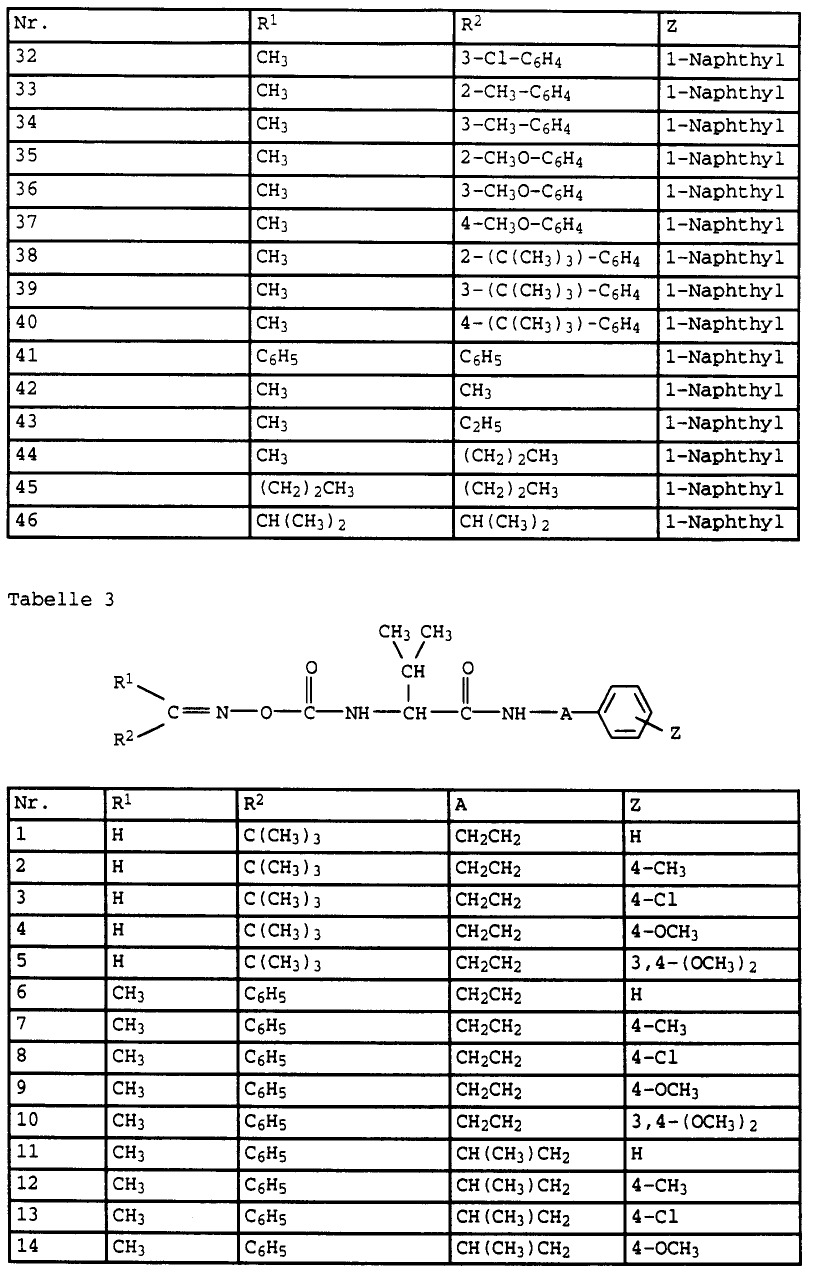

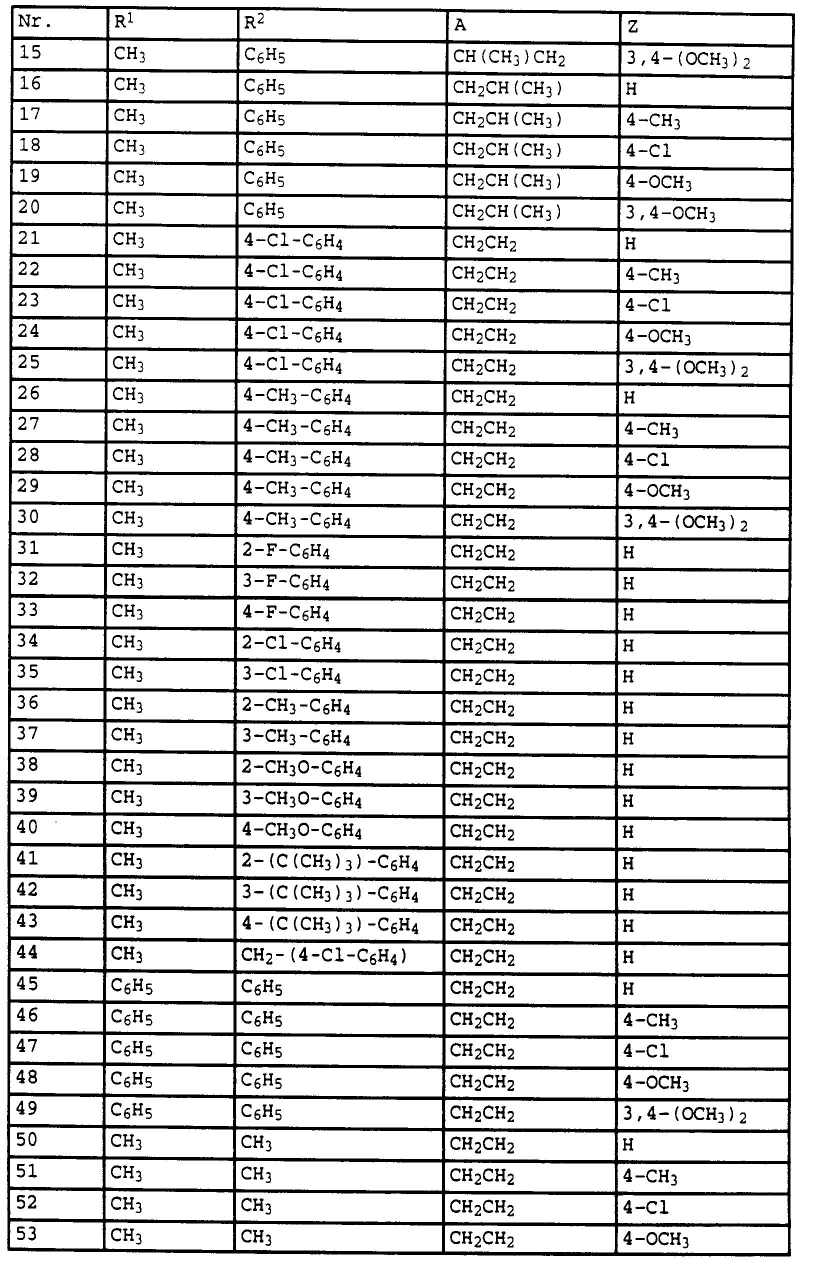

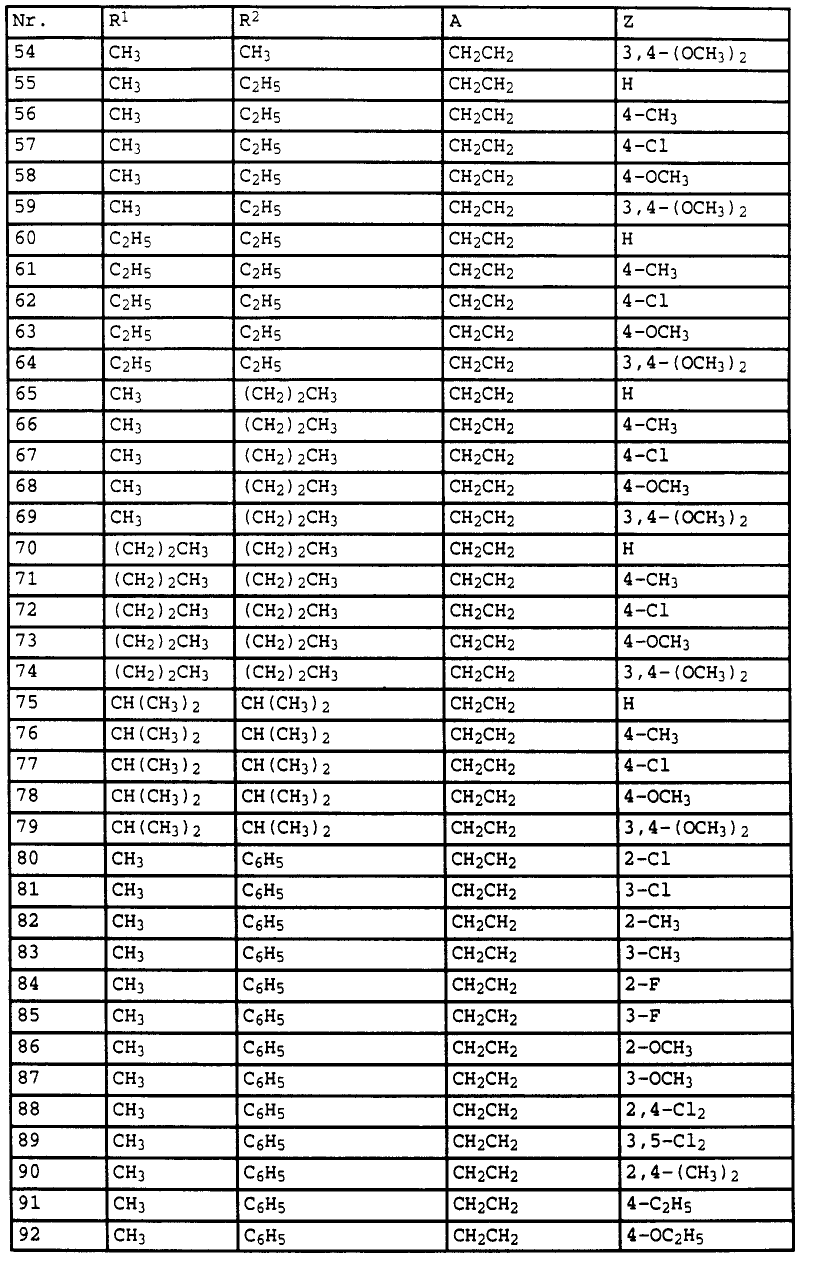

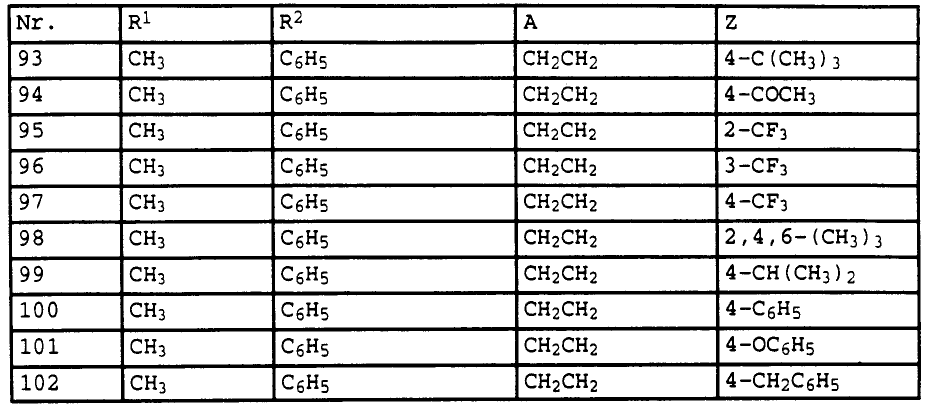

Die neuen Verbindungen der Formel I eignen sich als Fungizide.

Die neuen Verbindungen, bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethorvliertes iso-Octyl-, Octyl- oder Nonylphenol, Alkylphenol-, Tributylphenylpolyglykolether, Alkylarylpolyetheralkohole, iso-Tridecylalkohol, Fettalkoholethylenoxid-Kondensate, ethoryliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung Nr. 3 aus Tabelle 4, und 10 Gew.-Teilen N-Methyl-2-pyrrolidon, die zur Anwendung in Form kleinster Tropfen geeignet ist;
II. eine Mischung aus 10 Gew.-Teilen der Verbindung Nr. 4 aus Tabelle 4, 70 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 10 Gew.-Teilen der Verbindung Nr. 5 aus Tabelle 4, 40 Gew.-Teilen Cyclohexanon, 30 Gew.-Teilen iso-Butanol, 20 Gew.-Teilen des Anlagerungsproduktes von 40 mol Ethylenoxid an 1 mol Ricinusöl;
IV. eine wäßrige Dispersion aus 10 Gew.-Teilen der Verbindung Nr. 21 aus Tabelle 4, 25 Gew.-Teilen Cyclohexanol, 55 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 3 aus Tabelle 4, 3 Gew.-Teilen des Natriumsalzes der Di-iso-butylnaphthalin-2-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 4 aus Tabelle 4 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 21 aus Tabelle 4, 62 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 5 aus Tabelle 4, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 3 aus Tabelle 4, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 50 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Deuteromyceten, Ascomyceten, Phycomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die neuen Verbindungen zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,025 und 2, vorzugsweise 0,1 bis 1 kg Wirkstoff pro ha.

Bei der Saatgutbehandlung werden im allgemeinen Wirkstoffmengen von 0,001 bis 50, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid;
Nitroderivate, wie Dinitro-(1-methylheptyl)-phenylcrotonat, 2-sec.-Butyl-4,6-dinitrophenyl-3,3-dimethylacrylat, 2-sec.-Butyl-4,6-dinitrophenyl-iso-propylcarbonat, 5-Nitro-iso-phthalsäure-di-iso-propylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thion-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethylfuran-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan,
2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, (2-Chlorphenyl)-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, [2-(4-Chlorphenyl)ethyl]-(1,1-dimethylethyl)-1H-1,2,4-triazol-1-ethanol, 1-[3-(2-Chlorphenyl)-1-(4-fluorphenyl)oxiran-2-yl-methyl]-1H-1,2,4-triazol sowie
verschiedene Fungizide, wie Dodecylguanidinacetat, 3-[3-(3,5-Dimethyl-2-oxycyclohexyl)-2-hydroxyethyl]glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[(3,5-Dichlorphenyl)-5-methyl-5-methoxymethyl-1,3-oxazolidin-2,4-dion, 3-(3,5-Dichlorphenyl)-1-iso-propylcarbamoylhydantoin, N-(3,5-Dichlorphenyl)-1,2-dimethylcyclopropan1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol,
Strobilurine wie Methyl-E-methoximino-[α-(o-tolyloxy)-o-tolyl]acetat, Methyl-E-2-{2-[6-(2-cyanophenoxy)pyridimin-4-yl-oxy]phenyl}-3-methoxyacrylat, Methyl-E-methoximino-[α-(2,5-dimethyloxy)-o-tolyl]acetamid.

Anilino-Pyrimidine wie N-(4,6-dimethylpyrimidin-2-yl)anilin, N-[4-methyl-6-(1-propinyl)pyrimidin-2-yl]anilin, N-(4-methyl-6-cyclopropyl-pyrimidin-2-yl)anilin.

Phenylpyrrole wie 4-(2,2-difluor-1,3-benzodioxol-4-yl)-pyrrol-3-carbonitril.

Zimtsäureamide wie 3-(4-chlorphenyl)-3-(3,4-dimethoxyphenyl)acrylsäuremorpholid.

### Synthesebeispiele

Die in den nachstehenden Synthesebeispielen wiedergegebenen Vorschriften zur Herstellung der Verbindungen I können unter Abwandlung der Ausgangsverbindungen zur Gewinnung weiterer Vertreter der Verbindungen I benutzt werden. Die physikalischen Daten der demgemäß hergestellten Produkte sind in der anschließenden Tabelle 4 wiedergegeben.

### Beispiel 1

### N-(1-Phenylethyliden)-aminooxycarbonyl-L-valin-1-(4-methylphenyl)ethylamid

Zu 6,26 g (19 mmol) N-(tert.-Butyloxycarbonyl)-L-valin-1-(4-methylphenyl)ethylamid (Herstellung gemäß EP-A 398 072) wurden unter Kühlung 30 ml Trifluoressigsäure gegeben und die Mischung 1 Stunde bei 0°C gerührt. Danach erwärmte man auf 20°C, destillierte die Trifluoressigsäure weitgehend ab und nahm den Rückstand in Dichlormethan auf. Nach dem Waschen mit jeweils 40 ml 10 %-iger Natronlauge bzw. Wasser wurde die organische Phase getrocknet und das Lösungsmittel entfernt. Zurück blieben 3,05 g L-Valin-1-(4-methylphenyl)ethylamid in Form eines zähen Öls.

Zu 1 g (4,3 mmol) dieser Verbindung und 0,43 g (4,3 mmol) Triethylamin in 40 ml Toluol wurden bei 0°C 0,84 g (4,3 mmol) 1-Phenylethanon-O-chlorcarbonyloxim getropft. Es wurde 15 Stunden bei Raumtemperatur nachgerührt und die organische Phase danach mit jeweils 40 ml 10%-iger Salzsäure, 10%-iger Natriumhydrogencarbonat-Lösung bzw. Wasser gewaschen. Die organische Phase wurde getrocknet und eingeengt. Es verblieben 0,54 g der Titelverbindung als farbloser kristalliner Rückstand (Fp.: 110°C).

### Beispiel 2

### N-[1-(4-Chlorphenylethyliden)-aminooxycarbonyl]-L-valin-2-(3,4-dimethoxyphenyl)-ethylamid

Zu 7,6 g (0,02 mol) N-(tert.-Butyloxycarbonyl)-L-valin-2-(3,4-dimethoxyphenyl)ethylamid (Herstellung gemäß EP-A 0 493 683) wurden unter Kühlung 30 ml Trifluoressigsäure gegeben und die Mischung 1 Stunde bei 0°C nachgerührt. Danach wurde auf 20°C erwärmt, die Trifluoressigsäure weitgehend abdestilliert und der Rückstand in Dichlormethan aufgenommen. Nach dem Waschen mit jeweils 40 ml 10 %-iger Natronlauge bzw. Wasser wurde die organische Phase getrocknet und eingeengt. Es verblieben 3,2 g L-Valin-2-(3,4-dimethoxyphenyl)ethylamid.

Zu 1,4 g (5 mmol) dieser Verbindung und 0,52 g (5,2 mmol) Triethylamin in 40 ml Toluol wurden bei 0°C 1,16 g (5 mmol) 1-(4-Chlorphenyl)ethanon-O-chlorcarbonyloxim getropft. Nach 15 Stunden bei 20°C wurde die organische Phase nacheinander mit jeweils 40 ml 10%-iger Salzsäure, 10%-iger Natriumhydrogencarbonat-Lösung bzw. Wasser gewaschen. Anschließend wurde sie getrocknet und eingeengt. Es verblieben 1,49 g der Titelverbindung (Fp.: 105°C).

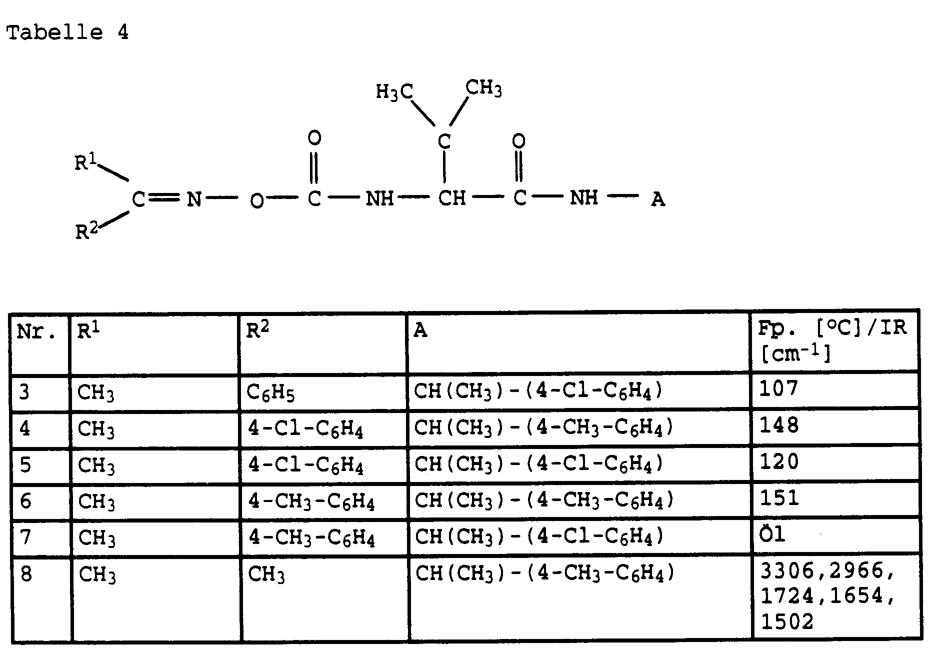

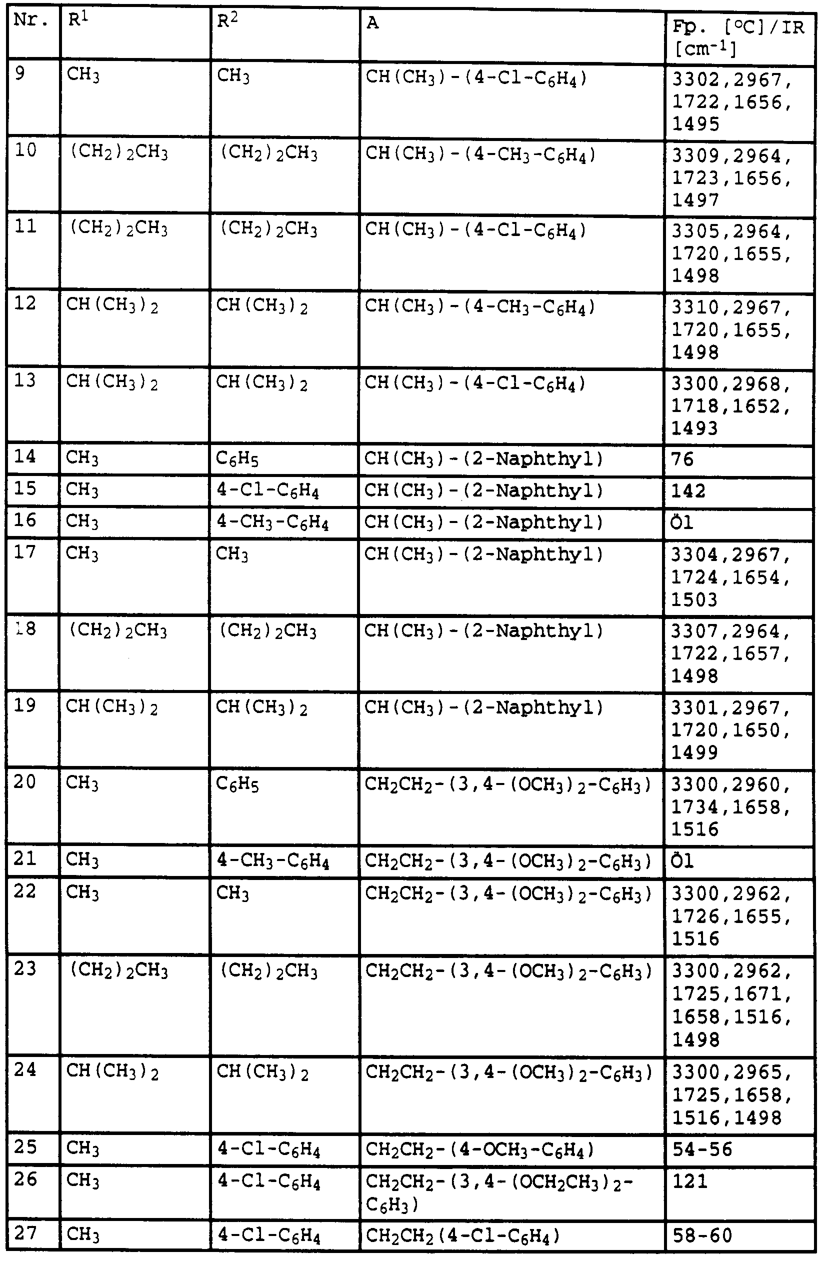

### Anwendungsbeispiele

Die fungizide Wirkung der Verbindungen der allgemeinen Formel I ließ sich durch folgende Versuche zeigen:
Die Wirkstoffe wurden als 20 gew.-%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Plasmopara viticola

Blätter von Topfreben der Sorte "Müller-Thurgau" wurden mit wäßriger Spritzbrühe, die 80 Gew.-% Wirkstoff und 20 Gew.-% Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Die Reben wurden zunächst für 48 Stunden in einer Kammer mit wasserdampf-gesättigter Luft bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die visuelle Beurteilung des Ausmaßes des Pilzausbruchs auf den Blattunterseiten.

In diesem Test zeigten jeweils die mit einer 250 ppm enthaltenden wäßrigen Aufbereitung der Verbindungen gemäß den Synthesebeispielen 1, 2, 3, 4, 5, 6, 7 bzw. 21 behandelten Pflanzen einen maximalen Befall von 5 %, während unbehandelte Pflanzen zu 65 % befallen waren.

### Phytophthora infestans

Blätter von Topfpflanzen der Sorte "Große Fleischtomate" wurden mit wäßriger Spritzbrühe, die 80 Gew.-% Wirkstoff und 20 Gew.-% Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer Kammer mit wasserdampfgesättigter Luft bei Temperaturen zwischen 16 und 18°C aufgestellt. Nach 6 Tagen hatte sich der Befall auf den unbehandelten, jedoch infizierten Kontrollpflanzen so stark entwickelt, daß die fungizide Wirksamkeit der Substanzen visuell beurteilt werden konnte.

In diesem Test zeigten jeweils die mit einer 250 ppm enthaltenden wäßrigen Aufbereitung der Verbindungen gemäß den Synthesebeispielen 1, 2, 3, 5, 6, 7, 10, 11, 13, 19, 21, 22, 23 bzw. 24 behandelten Pflanzen einen maximalen Befall von 15 %, während unbehandelte Pflanzen zu 75 % befallen waren.

## Patentansprüche

1. Carbamoyloximcarbonsäureamide der allgemeinen Formel I 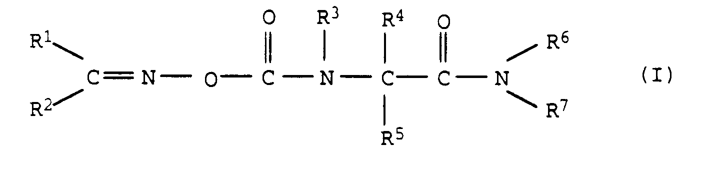 sowie deren Salze, in denen die Reste R¹ bis R⁷ die folgende Bedeutung haben:
R¹ C₁-C₈-Alkyl, wobei diese Reste partiell oder vollständig halogeniert sein und/oder eine oder zwei der folgenden Gruppen tragen können: C₁-C₄-Alkoxy, C₁-C₄-Halogenalkoxy, C₁-C₄-Alkylthio, C₃-C₇-Cycloalkyl, C₅-C₇-Cycloalkenyl, Aryl und Heteroaryl, wobei die cyclischen Reste ihrerseits einen bis drei der folgenden Substituenten tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy,
C₂-C₈-Alkenyl oder C₂-C₈-Alkinyl, wobei diese Reste partiell oder vollständig halogeniert sein und/oder eine oder zwei der folgenden Gruppen tragen können: C₁-C₄-Halogenalkyl, C₁-C₄-Alkoxyalkyl, C₁-C₄-Alkoxy, C₁-C₄-Halogenalkoxy, Aryl und Aryloxy,
C₃-C₇-Cycloalkyl oder C₅-C₇-Cycloalkenyl, wobei diese Reste partiell oder vollständig halogeniert sein und/oder eine oder zwei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio, Aryl und Aryl-(C₁-C₄)-alkyl, wobei in den Gruppen, welche Aryl enthalten dieses einen bis drei der folgenden Substituenten tragen kann: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy,
Aryl oder Heteroaryl, wobei diese Reste ein bis fünf Halogenatome und/oder eine bis drei der folgenden Gruppen tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy;
R² Wasserstoff oder einen der Reste R¹ oder
R¹ und R² gemeinsam mit dem Kohlenstoffatom das sie trägt einen Fluorenylrest, welcher eine bis drei der folgenden Gruppen tragen kann: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy oder
R¹ und R² gemeinsam mit dem Kohlenstoffatom das sie trägt einen 5- bis 8-gliedrigen gesättigten Ring, welcher als Ringglieder neben Kohlenstoff eines oder zwei der Heteroatome Sauerstoff, Schwefel und Stickstoff enthalten kann, wobei die Kohlenstoffatome im Ring eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy und wobei Stickstoffatome als Heteroatome Wasserstoff oder eine C₁-C₄-Alkylgruppe tragen;
R³ Wasserstoff, C₁-C₈-Alkyl oder C₃-C₇-Cycloalkyl, wobei diese Reste partiell oder vollständig halogeniert sein können;
R⁴ C₁-C₈-Alkyl, C₃-C₇-Cycloalkyl oder Phenyl-(C₁-C₄)-alkyl, wobei der Phenylrest ein bis fünf Halogenatome und/oder eine bis drei der folgenden Gruppen tragen kann: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy;
R⁵ Wasserstoff oder einen Rest R⁴ oder
R⁴ und R⁵ gemeinsam mit dem C-Atom an das sie gebunden sind einen 3- bis 8-gliedrigen Ring, welcher neben Kohlenstoff als Ringglieder eines oder zwei der Heteroatome Sauerstoff, Schwefel und Stickstoff enthalten kann, wobei die Kohlenstoffatome des Rings eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy und C₁-C₄-Halogenalkoxy und wobei Stickstoffatome als Heteroatome Wasserstoff oder eine C₁-C₄-Alkylgruppe tragen;
R⁶ Wasserstoff oder partiell oder vollständig halogeniertes C₁-C₈-Alkyl oder C₃-C₇-Cycloalkyl;
R⁷ C₁-C₈-Alkyl oder C₂-C₈-Alkenyl, wobei diese Reste partiell oder vollständig halogeniert sein und/oder eine oder zwei der folgenden Gruppen tragen können: Cyano, Nitro, C₁-C₄-Alkoxyalkyl, C₁-C₄-Halogenalkyl, C₁-C₄-Alkoxy, C₁-C₄-Halogenalkoxy, C₁-C₄-Alkylthio, C₁-C₄-Alkoxycarbonyl und Aryl und wobei die Arylgruppen ihrerseits einen bis drei der folgenden Substituenten tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Aryloxy,
C₃-C₇-Cycloalkyl oder C₅-C₇-Cycloalkenyl, wobei diese Reste partiell oder vollständig halogeniert sein, und/oder eine oder zwei der folgenden Gruppen tragen können: C₁-C₄-Alkyl, C₁-C₄-Halogenalkyl, C₁-C₄-Alkoxy, C₁-C₄-Halogenalkoxy, C₁-C₄-Alkylthio,
Aryl oder Heteroaryl, wobei diese Reste partiell oder vollständig halogeniert sein und/oder eine bis drei der folgenden Gruppen tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy oder
R⁶ und R⁷ gemeinsam mit dem Stickstoffatom an das sie gebunden sind einen 4- bis 8-gliedrigen gesättigten Ring, wobei der Ring noch eines oder zwei der Heteroatome Sauerstoff, Schwefel und Stickstoff enthalten kann und die Kohlenstoffatome des Rings eine oder zwei der folgenden Gruppen tragen können: Halogen, Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, Aryl und Heteroaryl und wobei die cyclischen Gruppen ein bis fünf Halogenatome und/oder einen bis drei der folgenden Substituenten tragen können: Cyano, Nitro, C₁-C₄-Alkyl, C₁-C₄-Alkoxyalkyl, C₁-C₄-Halogenalkyl, C₁-C₄-Alkoxy, C₁-C₄-Halogenalkoxy, C₁-C₄-Alkoxycarbonyl, Aryl und Aryloxy und wobei weitere Stickstoffatome als Heteroatome Wasserstoff oder eine C₁-C₄-Alkylgruppe tragen.

2. Verfahren zur Herstellung von Carbamoyloximcarbonsäureamiden der allgemeinen Formel I gemäß Anspruch 1, dadurch gekennzeichnet, daß man
a) aus einer N-geschützten Aminosäure der allgemeinen Formel IIa 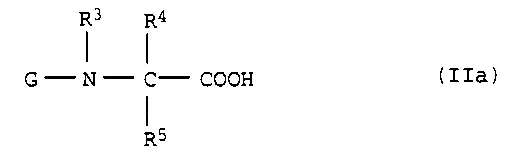 in der G eine an sich bekannte Aminosäure-Schutzgruppe bedeutet, und einem Amin der allgemeinen Formel III 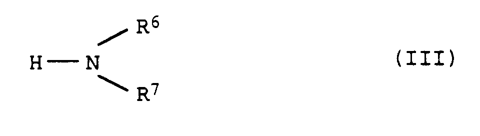 ein N-geschütztes Aminosäureamid der allgemeinen Formel IV herstellt 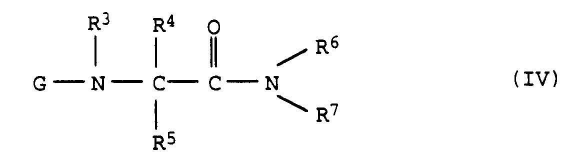
b) aus dem so erhaltenen N-geschützten Aminosäureamid IV durch Abspalten der Schutzgruppe G das Aminosäureamid der allgemeinen Formel V 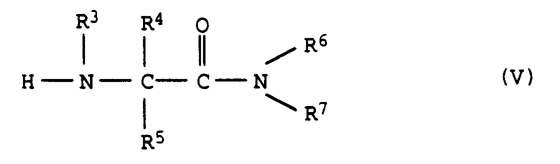 freisetzt und
c) das dabei anfallende Aminosäureamid V mit einem Chlorformyloxim der allgemeinen Formel VI 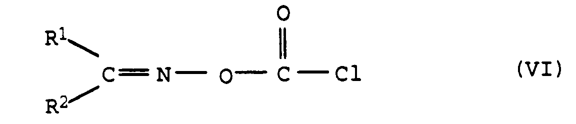 in Gegenwart einer Base umsetzt.

3. Verfahren zur Herstellung von Carbamoyloximcarbonsäureamiden der allgemeinen Formel I gemäß Anspruch 1, dadurch gekennzeichnet, daß man
a) eine Aminosäure der allgemeinen Formel II 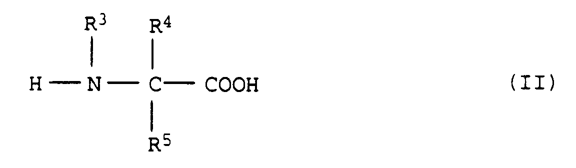 in Gegenwart einer Base mit einem Chlorformyloxim der allgemeinen Formel VI gemäß Anspruch 2 zu einer Carbamoyloximcarbonsäure der allgemeinen Formel VII 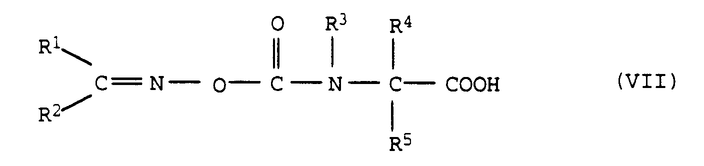 umsetzt und
b) die so erhaltene Carbamoyloximcarbonsäure VII mit einem Amin der allgemeinen Formel III nach Anspruch 2 zur Reaktion bringt.

4. Zur Bekämpfung von Schadpilzen geeignete Mittel, enthaltend übliche Zusatzstoffe und eine wirksame Menge einer Verbindung der allgemeinen Formel I gemäß Anspruch 1.

5. Verfahren zur Bekämpfung von Schadpilzen, dadurch gekennzeichnet, daß man die Schadpilze, deren Lebensraum oder die von ihnen freizuhaltenden Pflanzen, Flächen, Materialien oder Räume mit einer wirksamen Menge einer Verbindung der allgemeinen Formel I gemäß Anspruch 1 oder einem I enthaltenden Mittel gemäß Anspruch 4 behandelt.

6. Verwendung der Verbindungen der allgemeinen Formel I gemäß Anspruch 1 zur Bekämpfung von Schadpilzen.

## Claims

1. A carbamoyloximecarboxamide of the general formula I 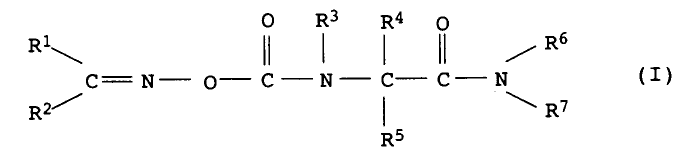 or its salts, where the radicals R¹ to R⁷ have the following meanings:
R¹ is C₁-C₈-alkyl, these radicals being partly or completely halogenated and/or being able to carry one or two of the following groups: C₁-C₄-alkoxy, C₁-C₄-haloalkoxy, C₁-C₄-alkylthio, C₃-C₇-cycloalkyl, C₅-C₇-cycloalkenyl, aryl and heteroaryl, the cyclic radicals for their part being able to carry one to three of the following substituents: halogen, cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy,
C₂-C₈-alkenyl or C₂-C₈-alkynyl, these radicals being partly or completely halogenated and/or being able to carry one or two of the following groups: C₁-C₄-haloalkyl, C₁-C₄-alkoxyalkyl, C₁-C₄-alkoxy, C₁-C₄-haloalkoxy, aryl and aryloxy,
C₃-C₇-cycloalkyl or C₅-C₇-cycloalkenyl, these radicals being partly or completely halogenated and/or being able to carry one or two of the following groups: C₁-C₄-alkyl, C₁-C₄-haloalkyl, C₁-C₄-alkoxy, C₁-C₄-haloalkoxy, C₁-C₄-alkylthio, aryl and aryl-(C₁-C₄)-alkyl, in the groups which contain aryl this being able to carry one to three of the following substituents: halogen, cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy,
aryl or heteroaryl, these radicals being able to carry one to five halogen atoms and/or one to three of the following groups: cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy;
R² is hydrogen or one of the radicals R¹ or
R¹ and R², together with the carbon atom which carries them, are a fluorenyl radical which can carry one to three of the following groups: halogen, cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy or
R¹ and R², together with the carbon atom which carries them, are a 5- to 8-membered saturated ring which, as ring members, in addition to carbon can contain one or two of the heteroatoms oxygen, sulfur and nitrogen, the carbon atoms in the ring being able to carry one or two of the following groups: halogen, cyano, nitro, C₁-C₄-alkyl, C₁-C₄-alkoxyalkyl, C₁-C₄-haloalkyl, C₁-C₄-alkoxy, C₁-C₄-haloalkoxy, aryl and aryloxy, and nitrogen atoms as heteroatoms carrying hydrogen or a C₁-C₄-alkyl group;
R³ is hydrogen, C₁-C₈-alkyl or C₃-C₇-cycloalkyl, where these radicals can be partly or completely halogenated;
R⁴ is C₁-C₈-alkyl, C₃-C₇-cycloalkyl or phenyl-(C₁-C₄)alkyl, the phenyl radical being able to carry one to five halogen atoms and/or one to three of the following groups: cyano, nitro, C₁-C₄-alkyl, C₁-C₄-alkoxyalkyl, C₁-C₄-haloalkyl, C₁-C₄-alkoxy, C₁-C₄-haloalkoxy, aryl and aryloxy;
R⁵ is hydrogen or a radical R⁴ or
R⁴ and R^{5,} together with the C atom to which they are bonded, are a 3- to 8-membered ring which, in addition to carbon, can contain one or two of the heteroatoms oxygen, sulfur and nitrogen as ring members, the carbon atoms of the ring being able to carry one or two of the following groups: halogen, cyano, nitro, C₁-C₄-alkyl, C₁-C₄-alkoxyalkyl, C₁-C₄-haloalkyl, C₁-C₄-alkoxy and C₁-C₄-haloalkoxy, and nitrogen atoms as heteroatoms carrying hydrogen or a C₁-C₄-alkyl group;
R⁶ is hydrogen or partly or completely halogenated C₁-C₈-alkyl or C₃-C₇-cycloalkyl;
R⁷ is C₁-C₈-alkyl or C₂-C₈-alkenyl, these radicals being partly or completely halogenated and/or being able to carry one or two of the following groups: cyano, nitro, C₁-C₄-alkoxyalkyl, C₁-C₄-haloalkyl, C₁-C₄-alkoxy, C₁-C₄-haloalkoxy, C₁-C₄-alkylthio, C₁-C₄-alkoxycarbonyl and aryl, and the aryl groups for their part being able to carry one to three of the following substituents: halogen, cyano, nitro, C₁-C₄-alkyl, C₁-C₄-alkoxyalkyl, C₁-C₄-haloalkyl, C₁-C₄-alkoxy, C₁-C₄-haloalkoxy, aryl and aryloxy,
C₃-C₇-cycloalkyl or C₅-C₇-cycloalkenyl, these radicals being partly or completely halogenated, and/or being able to carry one or two of the following groups: C₁-C₄-alkyl, C₁-C₄-haloalkyl, C₁-C₄-alkoxy, C₁-C₄-haloalkoxy, C₁-C₄-alkylthio,
aryl or heteroaryl, these radicals being partly or completely halogenated and/or being able to carry one to three of the following groups: cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy or
R⁶ and R⁷, together with the nitrogen atom to which they are bonded, are a 4- to 8-membered saturated ring, the ring additionally being able to contain one or two of the heteroatoms oxygen, sulfur and nitrogen and the carbon atoms of the ring being able to carry one or two of the following groups: halogen, cyano, nitro, C₁-C₄-alkyl, C₁-C₄-alkoxyalkyl, C₁-C₄-haloalkyl, C₁-C₄-alkoxy, C₁-C₄-haloalkoxy, aryl and heteroaryl, and the cyclic groups being able to carry one to five halogen atoms and/or one to three of the following substituents: cyano, nitro, C₁-C₄-alkyl, C₁-C₄-alkoxyalkyl, C₁-C₄-haloalkyl, C₁-C₄-alkoxy, C₁-C₄-haloalkoxy, C₁-C₄-alkoxycarbonyl, aryl and aryloxy, and further nitrogen atoms as heteroatoms carrying hydrogen or a C₁-C₄-alkyl group.

2. A process for preparing carbamoyloximecarboxamides of the general formula I as claimed in claim 1, which comprises
a) from an N-protected amino acid of the general formula IIa 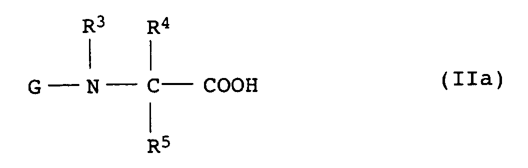 where G is an amino acid protective group known per se, and an amine of the general formula III 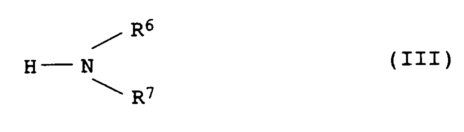 preparing an N-protected amino acid amide of the general formula IV 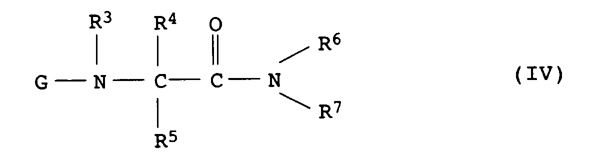
b) from the N-protected amino acid amide IV thus obtained liberating the amino acid amide of the general formula V 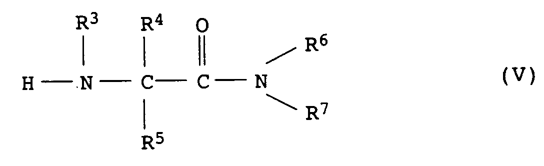 by removing the protective group G and
c) reacting the amino acid amide V obtained in this process with a chloroformyl oxime of the general formula VI 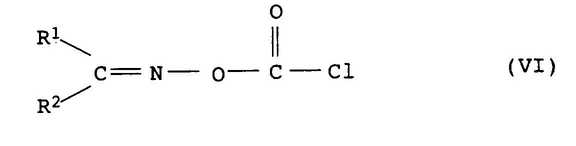 in the presence of a base.

3. A process for preparing carbamoyloximecarboxamides of the general formula I as claimed in claim 1, which comprises
a) reacting an amino acid of the general formula II 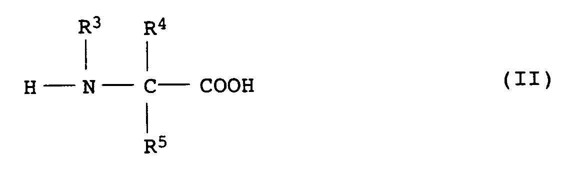 in the presence of a base with a chloroformyl oxime of the general formula VI as claimed in claim 2 to give a carbamoyloximecarboxylic acid of the general formula VII 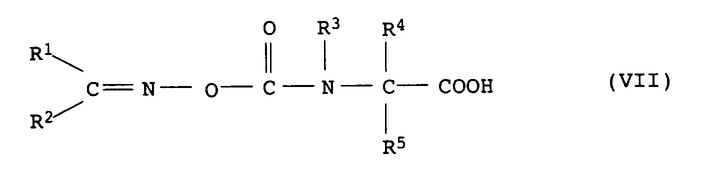 and
b) reacting the carbamoyloximecarboxylic acid VII thus obtained with an amine of the general formula III as in claim 2.

4. A composition suitable for controlling harmful fungi, containing customary additives and an effective amount of a compound of the general formula I as claimed in claim 1.

5. A process for controlling harmful fungi, which comprises treating the harmful fungi, their environment or the plants, areas, materials or spaces to be kept free from them with an effective amount of a compound of the general formula I as claimed in claim 1 or a composition containing I as claimed in claim 4.

6. The use of the compounds of the general formula I as claimed in claim 1 for controlling harmful fungi.

## Revendications

1. Amides d'acides carbamoyloximecarboxyliques de formule générale I 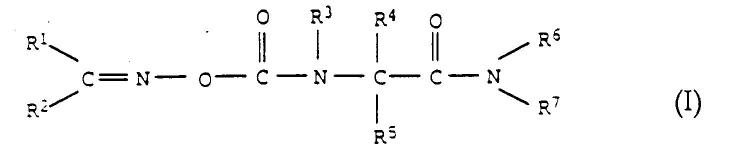 et leurs sels, pour lesquels les symboles R¹ à R⁷ ont les significations suivantes :
R¹ : un groupe alkyle en C1-C8 qui peut être partiellement ou totalement halogéné et/ou peut porter un ou deux des substituants suivants : alcoxy en C1-C4, halogénoalcoxy en C1-C4, alkylthio en C1-C4, cycloalkyle en C3-C7, cycloalcényle en C5-C7, aryle et hétéroaryle, les substituants cycliques pouvant eux-mêmes porter un à trois des substituants suivants : halogéno, cyano, nitro, alkyle en C1-C4, (alcoxy en C1-C4)alkyle, halogénoalkyle en C1-C4, alcoxy en C1-C4, halogénoalcoxy en C1-C4, (alcoxy en C1-C4)-carbonyle, aryle et aryloxy,
un groupe alcényle en C2-C8 ou alcynyle en C2-C8 qui peut être partiellement ou totalement halogéné et/ou peut porter un ou deux des groupes suivants : halogénoalkyle en C1-C4, (alcoxy en C1-C4)alkyle, alcoxy en C1-C4, halogénoalcoxy en C1-C4, aryle et aryloxy,
un groupe cycloalkyle en C3-C7 ou cycloalcényle en C5-C7 qui peut être partiellement ou totalement halogéné et/ou peut porter un ou deux des groupes suivants : alkyle en C1-C4, halogénoalkyle en C1-C4, alcoxy en C1-C4, halogénoalcoxy en C1-C4, alkylthio en C1-C4, aryle et aryl-alkyle en C1-C4, les parties aryles de ces derniers groupes pouvant porter un à trois des substituants suivants : halogéno, cyano, nitro, alkyle en C1-C4, (alcoxy en C1-C4)alkyle, halogénoalkyle en C1-C4, alcoxy en C1-C4, halogéno-alcoxy en C1-C4, (alcoxy en C1-C4)carbonyle, aryle et aryloxy,
un groupe aryle ou hétéroaryle, qui peut porter un à cinq atomes d'halogènes et/ou un à trois des groupes suivants : cyano, nitro, alkyle en C1-C4, (alcoxy en C1-C4)-alkyle, halogénoalkyle en C1-C4, alcoxy en C1-C4, halogénoalcoxy en C1-C4, (alcoxy en C1-C4)carbonyle, aryle et aryloxy;
R² représente l'hydrogène ou a l'une des significations de R¹ ou bien
R¹ et R² forment ensemble et avec l'atome de carbone qui les porte un groupe fluorényle qui peut porter un à trois des substituants suivants : halogéno, cyano, nitro, alkyle en C1-C4, alcoxy en C1-C4, halogénoalkyle en C1-C4, alcoxyalkyle en C1-C4, halogénoalcoxy en C1-C4, (alcoxy en C1-C4)carbonyle, aryle et aryloxy, ou bien
R¹ et R² forment ensemble et avec l'atome de carbone qui les porte un cycle saturé de cinq à huit chaînons qui peut contenir en tant que chaînons cycliques, en plus du carbone, un ou deux des hétéroatomes oxygène, soufre et azote, les atomes de carbone du cycle pouvant porter un ou deux des substituants suivants : halogéno, cyano, nitro, alkyle en C1-C4, alcoxyalkyle en C1-C4, halogénoalkyle en C1-C4, alcoxy en C1-C4, halogénoalcoxy en C1-C4, aryle et aryloxy, les hétéroatomes d'azote pouvant porter de l'hydrogène ou un groupe alkyle en C1-C4;
R³ représente l'hydrogène, un groupe alkyle en C1-C8 ou cycloalkyle en C3-C7, ces groupes pouvant être partiellement ou totalement halogénés;
R⁴ représente un groupe alkyle en C1-C8, cycloalkyle en C3-C7 ou phényl-alkyle en C1-C4, la partie phényle pouvant porter un à cinq atomes d'halogènes et/ou un à trois des substituants suivants : cyano, nitro, alkyle en C1-C4, alcoxyalkyle en C1-C4, halogénoalkyle en C1-C4, alcoxy en C1-C4, halogénoalcoxy en C1-C4, aryle et aryloxy;
R⁵ représente l'hydrogène ou a l'une des significations de R⁴, ou bien
R⁴ et R⁵ forment ensemble et avec l'atome de carbone qui les porte un cycle de trois à huit chaînons qui peut contenir en tant que chaînons cycliques, en plus de carbone, un ou deux des hétéroatomes oxygène, soufre et azote, les atomes de carbone cycliques pouvant porter un ou deux des substituants suivants : halogéno, cyano, nitro, alkyle en C1-C4, alcoxyalkyle en C1-C4, halogénoalkyle en C1-C4, alcoxy en C1-C4 et halogénoalcoxy en C1-C4, les hétéroatomes d'azote pouvant porter l'hydrogène ou un groupe alkyle en C1-C4;
R⁶ représente l'hydrogène ou un groupe alkyle en C1-C8 ou cycloalkyle en C3-C7, partiellement ou totalement halogéné;
R⁷ représente un groupe alkyle en C1-C8 ou alcényle en C2-C8 qui peut être partiellement ou totalement halogéné et/ou peut porter un ou deux des groupes suivants : cyano, nitro, alcoxyalkyle en C1-C4, halogénoalkyle en C1-C4, alcoxy en C1-C4, halogénoalcoxy en C1-C4, alkylthio en C1-C4, (alcoxy en C1-C4)carbonyle et aryle, les groupes aryle pouvant eux-mêmes porter un à trois des substituants suivants : halogéno, cyano, nitro, alkyle en C1-C4, alcoxyalkyle en C1-C4, halogénoalkyle en C1-C4, alcoxy en C1-C4, halogénoalcoxy en C1-C4, aryle et aryloxy,
un groupe cycloalkyle en C3-C7 ou cycloalcényle en C5-C7 qui peut être partiellement ou totalement halogéné et/ou peut porter un ou deux des substituants suivants : alkyle en C1-C4, halogénoalkyle en C1-C4, alcoxy en C1-C4, halogénoalcoxy en C1-C4, alkylthio en C1-C4,
un groupe aryle ou hétéroaryle qui peut être partiellement ou totalement halogéné et/ou peut porter un à trois des substituants suivants : cyano, nitro, alkyle en C1-C4, alcoxyalkyle en C1-C4, halogénoalkyle en C1-C4, alcoxy en C1-C4, halogénoalcoxy en C1-C4, (alcoxy en C1-C4)carbonyle, aryle et aryloxy, ou bien
R⁶ et R⁷ forment ensemble et avec l'atome d'azote auquel ils sont reliés un cycle saturé de quatre à huit chaînons, ce cycle pouvant encore contenir un ou deux des hetéroatomes oxygène, soufre et azote et les atomes de carbone cycliques pouvant porter un ou deux des substituants suivants : halogéno, cyano, nitro, alkyle en C1-C4, alcoxyalkyle en C1-C4, halogénoalkyle en C1-C4, alcoxy en C1-C4, halogénoalcoxy en C1-C4, aryle et hétéroaryle, les groupes cycliques pouvant porter un à cinq atomes d'halogènes et/ou un à trois des substituants suivants : cyano, nitro, alkyle en C1-C4, alcoxyalkyle en C1-C4, halogénoalkyle en C1-C4, alcoxy en C1-C4, halogénoalcoxy en C1-C4, (alcoxy en C1-C4)carbonyle, aryle et aryloxy, les hétéroatomes d'azote pouvant porter l'hydrogène ou un groupe alkyle en C1-C4.

2. Procédé de préparation des amides d'acides carbamoyloximecarboxyliques de formule I selon la revendication 1, caractérisé par le fait que
**a)** à partir d'un aminoacide protégé à l'azote et répondant à la formule générale IIa 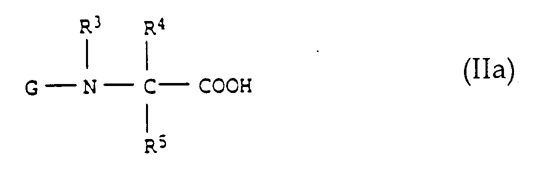 dans laquelle G représente un groupe protecteur pour aminoacides de type connu, et d'une amine de formule générale III 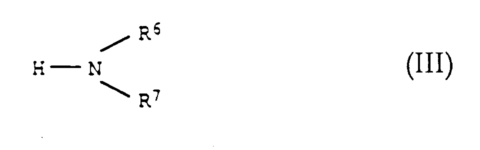 on prépare un amide d'aminoacide protégé à l'azote, répondant à la formule générale IV 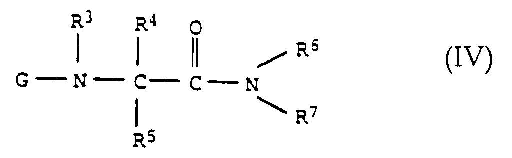
**b)** à partir de cet amide d'aminoacide protégé à l'azote IV, on libère par scission du groupe protecteur G l'amide d'aminoacide de formule générale V 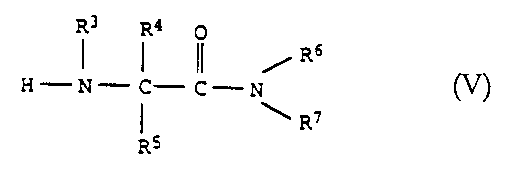 et
**c)** on fait réagir cet amide d'aminoacide V avec une chloroformyloxime de formule générale VI 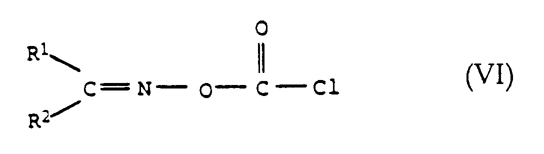 en présence d'une base.

3. Procédé de préparation des amides d'acide carbamoyloximecarboxyliques de formule générale I selon la revendication 1, caractérisé par le fait que
**a)** on fait réagir un aminoacide de formule générale II 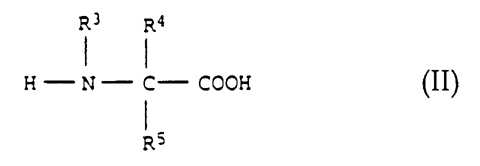 en présence d'une base, avec une chloroformyloxime de formule générale VI selon la revendication 2, ce qui donne un acide carbamoyloximecarboxylique de formule générale VII 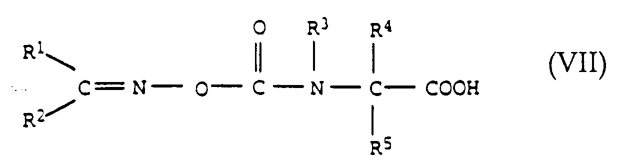 et
**b)** on fait réagir cet acide carbamoyloximecarboxylique VII avec une amine de formule générale III selon la revendication 2.

4. Produit approprié à la lutte contre les mycètes nuisibles, contenant des additifs usuels et une quantité efficace d'un composé de formule générale I selon la revendication 1.

5. Procédé pour combattre les mycètes nuisibles, caractérisé par le fait que l'on traite les mycètes nuisibles, leur habitat ou les végétaux, aires, matériaux ou locaux à protéger par une quantité efficace d'un composé de formule générale I selon la revendication 1 ou d'un produit contenant I selon la revendication 4.

6. Utilisation des composés de formule générale I selon la revendication 1 pour la lutte contre les mycètes nuisibles.
